# EUROPEAN PATENT APPLICATION

(11) **EP 3 950 928 A1**
(43) Date of publication of application: **09.02.2022**
(21) Application number: 20778494.3
(22) Date of filing: 20.03.2020
(51) Int. Cl.: C12N 1/20, A23K 10/16, A23C 9/12, A23L 33/135, A61K 35/747, A61P 19/02, A61P 29/00, C12R 1/225

(54) **KIMCHI LACTIC ACID BACTERIA LACTOBACILLUS SAKEI WIKIM0109 HAVING EFFICACY FOR RELIEF OF ARTHRITIS**

(30) Priority: 26.03.2019 KR 20190034211
(71) Applicant: Korea Food Research Institute, Wanju-gun, Jeollabuk-do 55365 (KR)
(72) Inventor: CHOI, Hak Jong, Gwangju 62065 (KR); KWON, Min Sung, Gwangju 61052 (KR)
(74) Representative: V.O.
(86) International application number: PCT/KR2020/003883
(87) International publication number: WO 2020/197188

(57) **Abstract**

The present disclosure relates to novel *Lactobacillus sakei* WIKIM0109 isolated from kimchi and a composition containing the same. The *Lactobacillus sakei* WIKIM0109 according to the present disclosure is a lactic acid bacterium having an activity of inhibiting IgG and IgG2a production in blood and an activity of inhibiting IFN-_{Y}, IL-17 and TNF-α production, and can be used in various applications, such as for preventing and ameliorating arthritis or improving intestinal regulation in human or animals.

## Description

### [Technical Field]

The present disclosure relates to a novel *Lactobacillus sakei* strain and a composition containing the same.

### [Background Art]

Rheumatoid arthritis is an autoimmune disease and chronic inflammatory disease with a prevalence rate of 1-1.5%. It induces joint swelling and pain due to inflammatory changes in the joint induce systemic and, when aggravated, may cause severe results such as deformity of the joint, difficulty in joint flexion, etc.

The direct cause of arthritis has not been clearly understood yet, and steroids, nonsteroidal anti-inflammatory drugs, anti-gout drugs, immunosuppressants, etc. are used as therapeutic agents for treating arthritis. However, these therapeutic agents do not provide ultimate therapeutic effects and their use is limited due to various side effects. In addition, some chemotherapeutic medications have weaknesses such as insufficient efficacy for arthritis that has occurred already.

Accordingly, development of a therapeutic agent for arthritis which is effective in relieving inflammatory symptoms and pain with few side effects and can be medicated conveniently is necessary. The inventors of the present disclosure have researched on a kimchi-derived lactic acid bacterium which effectively relieves the symptoms of arthritis and inhibits the production of inflammation-related factors in blood.

### [References of Related Art]

### [Patent Documents]

(Patent document 1) Korean Patent Publication No. 10-2018-0104544.

### [Disclosure]

### [Technical Problem]

The present disclosure is directed to providing a novel lactic acid bacterium in the genus *Lactobacillus* which has superior activity of ameliorating arthritis.

### [Technical Solution]

The inventors of the present disclosure have made efforts to find a strain exhibiting an effect of ameliorating arthritis. As a result, they have isolated and identified *Lactobacillus sakei* WIKIM0109, which is a novel lactic acid bacterium strain in the genus *Lactobacillus,* and have completed the present disclosure.

The *Lactobacillus sakei* WIKIM0109 according to the present disclosure is a novel *Lactobacillus sakei* strain derived from kimchi. Although it is described in the present disclosure that the *Lactobacillus sakei* WIKIM0109 is isolated and identified from kimchi, it can also be obtained from other sources.

As a result of 16S rRNA base sequencing for identification and classification of the lactic acid bacterium strain isolated from the traditional fermented food in an example of the present disclosure, it has been found to have a nucleic acid sequence of SEQ ID NO: 1.

Therefore, the microorganism of the present disclosure having the 16S rRNA base sequence of SEQ ID NO: 1 was named *Lactobacillus sakei* WIKIM0109 and was deposited in the Korean Collection for Type Cultures on March 7, 2019 (accession number: KCTC13818BP).

The *Lactobacillus sakei* WIKIM0109 (accession number: KCTC13818BP) strain of the present disclosure is a Gram-positive bacterium. It is a rod-shaped facultative anaerobe that can grow under both aerobic and anaerobic conditions.

The *Lactobacillus sakei* WIKIM0109 of the present disclosure has the effect of intestinal regulation and immune enhancement of common lactic acid bacteria. It is well known that the lactic acid bacteria in the genus *Lactobacillus* have the effect of intestinal regulation and immune enhancement.

In an example which will be described below, it was confirmed that the *Lactobacillus sakei* WIKIM0109 strain of the present disclosure exhibits the effect of ameliorating arthritis by inhibiting IgG and IgG2a production in blood and inhibiting IFN-y, IL-17 and TNF-α production. Accordingly, the *Lactobacillus sakei* WIKIM0109 according to the present disclosure can be used variously for intestinal regulation, amelioration of arthritis, immune enhancement, etc. in human or animals.

In an exemplary embodiment, the present disclosure provides a composition containing the *Lactobacillus sakei* WIKIM0109, a culture thereof, a lysate thereof or an extract thereof as an active ingredient.

The *Lactobacillus sakei* WIKIM0109 contained in the composition according to the present disclosure may be in the form of a living bacterium or in the form of a dried or freeze-dried bacterium. The types of lactic acid bacteria suitable for inclusion in various compositions and methods for preparing the same are well known to those skilled in the art.

In a specific exemplary embodiment, the composition may be a composition for oral administration, which contains the *Lactobacillus sakei* WIKIM0109 strain in the form of a living bacterium.

In another exemplary embodiment, the present disclosure provides a composition for intestinal regulation, which contains the *Lactobacillus sakei* WIKIM0109, a culture thereof, a lysate thereof or an extract thereof as an active ingredient. The composition according to the present disclosure for intestinal regulation can be used for preventing, treating or ameliorating gastrointestinal disease of animals including human. Specifically, the animals include livestock such as cow, horse and pig. The 'gastrointestinal disease' includes infection by gastrointestinal harmful bacteria and inflammatory bowel disease. For example, it includes infectious diarrhea caused by pathogenic microorganisms (*E*. *coli, Salmonella, Clostridium,* etc.), gastroenteritis, inflammatory bowel disease, neurological enteritis syndrome, microorganism overgrowth in the small intestine, intestinal acute diarrhea, etc., although not being limited thereto.

Specifically, the composition according to the present disclosure for intestinal regulation may be administered orally. The administration dosage may vary depending on the type of the gastrointestinal disease, the severity of the disease, age, sex, race, purpose such as treatment or prevention, etc. In general, 10 million to 100 billion bacteria may be administered per day for an adult.

The present disclosure provides a composition for enhancing immunity, which contains the *Lactobacillus sakei* WIKIM0109, a culture thereof, a lysate thereof or an extract thereof as an active ingredient. It is well known that the lactic acid bacteria in the genus *Lactobacillus* have the effect of enhancing immunity.

The present disclosure provides a composition for preventing and ameliorating arthritis, which contains the *Lactobacillus sakei* WIKIM0109, a culture thereof, a lysate thereof or an extract thereof as an active ingredient. The arthritis may be one or more disease selected from a group consisting of rheumatoid arthritis, ankylosing spondylitis, osteoarthritis or psoriatic arthritis, although not being limited thereto.

In a specific exemplary embodiment, the arthritis may be rheumatoid arthritis.

Because of these advantageous activities, the *Lactobacillus sakei* WIKIM0109 according to the present disclosure may be included in drugs, functional health foods, foods, feeds, feed additives or lactic acid bacterium starters for fermentation of foods or feeds.

In a specific exemplary embodiment, the composition of the present disclosure is used as a pharmaceutical composition, the pharmaceutical composition of the present disclosure may be prepared using a pharmaceutically suitable and physiologically acceptable adjuvant in addition to the active ingredient. As the adjuvant, an excipient, a disintegrant, a sweetener, a binder, a coating agent, a swelling agent, a lubricant, a glidant, a flavorant, etc. may be used.

Specifically, the pharmaceutical composition may be formulated by including one or more pharmaceutically acceptable carrier in addition to the active ingredient described above.

For example, for formulation into a tablet or a capsule, the active ingredient may be combined with an oral, nontoxic, pharmaceutically acceptable inert carrier such as ethanol, glycerol, water, etc. In addition, if necessary or desired, a suitable binder, lubricant, disintegrant or colorant or a mixture thereof may also be included. Suitable binders include, but are not limited to, starch, gelatin, natural sugars such as glucose or β-lactose, corn sweetener, natural or synthetic gums such as acacia, tragacanth or sodium oleate, sodium stearate, magnesium stearate, sodium benzoate, sodium acetate, sodium chloride, etc. Disintegrants include, but are not limited to, starch, methyl cellulose, agar, bentonite, xanthan gum, etc. For a composition formulated into a liquid solution, as a pharmaceutically acceptable carrier, one or more of saline, sterile water, Ringer's solution, buffered saline, albumin injection, dextrose solution, maltodextrin solution, glycerol and ethanol, which are biocompatible and suitable for sterilization, may be used. If necessary, other common additives such as an antioxidant, a buffer, a bacteriostat, etc. may be added. In addition, a diluent, a dispersant, a surfactant, a binder and a lubricant may be further added to formulate an injectable formulation such as an aqueous solution, a suspension, an emulsion, etc., a pill, a capsule, a granule or a tablet.

Specifically, the formulation may be prepared depending on the particular disease or ingredients using the methods described in Remington's Pharmaceutical Science (Mack Publishing Company, Easton, PA).

In another specific exemplary embodiment, the present disclosure may provide a food composition for preventing and ameliorating arthritis, which contains the *Lactobacillus sakei* WIKIM0109, a culture thereof, a lysate thereof or an extract thereof as an active ingredient. The food composition may be in the form of a functional health food, a beverage, a bar, etc.

In the present disclosure, the food composition containing the strain as an active ingredient may be in the form of a beverage such as fermented milk, etc. Therefore, the present disclosure provides a lactic acid bacterium starter for fermenting foods or feeds, which contains the *Lactobacillus sakei* WIKIM0109, a culture thereof, a lysate thereof or an extract thereof as an active ingredient.

The food composition according to the present disclosure may be formulated in the same manner as the pharmaceutical composition and may be used as a functional food or may be added to various foods. Examples of the food to which the composition of the present disclosure may be added include beverages, vitamin complexes, dietary health supplements, etc.

The food composition of the present disclosure may contain ingredients commonly added when preparing foods. Examples include a protein, a carbohydrate, a fat, a nutrient, a seasoning agent and a flavorant. Examples of the carbohydrate include common sugars such as monosaccharides, e.g., glucose, fructose, etc., disaccharides, e.g., maltose, sucrose, etc., oligosaccharides and polysaccharides, e.g., dextrin, cyclodextrin, etc. and sugar alcohols such as xylitol, sorbitol, erythritol, etc. As the flavorants, natural flavorants [thaumatin, stevia extract (e.g., rebaudioside A, glycyrrhizin, etc.)] or synthetic flavorants (saccharin, aspartame, etc.) may be used. For example, when the food composition of the present disclosure is prepared into a drink or a beverage, it may further contain citric acid, fructose syrup, sugar, glucose, acetic acid, malic acid, fruit juice, various plant extracts, etc.

The composition according to the present disclosure may be used as a feed additive or a feed.

When used as a feed additive, the composition may be prepared into a solution with a high concentration of 20-90%, a powder or a granule. The feed additive may further contain one or more of an organic acid such as citric acid, fumaric acid, adipic acid, lactic acid, malic acid, etc., a phosphate such as sodium phosphate, potassium phosphate, acidic pyrophosphate, polyphosphate, etc. or a natural antioxidant such as polyphenol, catechin, α-tocopherol, rosemary extract, vitamin C, green tea extract, licorice extract, chitosan, tannic acid, phytic acid, etc. When used as a feed, the composition may be prepared into an ordinary feed and may further contain common feed ingredients.

The feed additive and the feed may further contain a grain, e.g., ground or crushed wheat, oats, barley, maize and rice, a vegetable protein feed, e.g., a feed containing rape, bean and sunflower as main ingredients, an animal protein feed, e.g., blood meal, meat meal, bone meal and fish meal, a sugar or dairy product, e.g., various powder milk and whey powder. In addition, a nutritional supplement, a digestion- and absorption-enhancing agent, a growth-promoting agent, etc. may be further contained.

The feed additive may be administered to an animal either or in combination with another feed additive in an edible carrier. Further, the feed additive may be mixed directly into an animal feed as a top dressing or may be administered easily to an animal as an oral dosage form separately from a feed. When the feed additive is administered separately from an animal feed, it can be prepared into an immediate-release or sustained-release formulation in combination with a pharmaceutically acceptable edible carrier well known in the art. The edible carrier may be either solid or liquid, e.g., corn starch, lactose, sucrose, soybean flake, peanut oil, olive oil, sesame oil and propylene glycol. When a solid carrier is used, the feed additive may be in the form of a tablet, a capsule, a powder, a troche, a sugar-coated tablet or a non-dispersed top dressing. When a liquid carrier is used, the feed additive may be in the form of a soft gelatin capsule, a syrup, a suspension, an emulsion or a solution.

Further, the feed additive and the feed may contain an adjuvant, e.g., a preservative, a stabilizer, a wetting agent, an emulsifying agent, a solubilization promoter, etc. The feed additive may be added to an animal feed by dipping, spraying or mixing.

The feed or feed additive of the present disclosure can be provided to various animals, including mammals, poultry and fish.

The mammal may include pig, cow, sheep, goat, laboratory rodents, pets (e.g., dog or cat), etc. The poultry may include chicken, turkey, duck, goose, pheasant, quail, etc., and the fish may include trout, etc., although not being limited thereto.

The amount of the *Lactobacillus sakei* WIKIM0109 strain contained in the composition according to the present disclosure may be about 10⁶-10¹² CFU/g, e.g., 10⁷-10¹¹ CFU/g or 10⁸-10¹⁰ CFU/g, based on a single dose. Specifically, the strain may be administered as living bacteria, and may be killed or attenuated before intake. Further, a sterilization process through heat treatment may be performed additionally when the composition is prepared using a culture supernatant, etc. The amount of the strain required to achieve a minimal effect and the daily dose thereof may vary depending on the physical or health condition of a patient. Generally, the daily dose can be about 10⁶-10¹² CFU/g, e.g., 10⁷-10¹¹ CFU/g or 10⁸-10¹⁰ CFU/g.

The advantages and features of the present disclosure and the methods of accomplishing the same will become apparent by the following examples described in detail. However, the present disclosure is not limited to the examples set forth below but may be embodied in many different forms. The examples are provided such that the disclosure of the present disclosure is complete and are provided to fully inform the scope of the present disclosure to those having ordinary knowledge in the art to which the present disclosure belongs. The scope of the present disclosure will only be defined by the appended claims.

### [Advantageous Effects]

Because the *Lactobacillus sakei* WIKIM0109 according to the present disclosure exhibits an activity of inhibiting IgG and IgG2a production in blood and inhibiting IFN-y, IL-17 and TNF-α production, it can be utilized variously as a lactic acid bacterium for intestinal regulation, immune enhancement, prevention or amelioration of gastrointestinal disease and arthritis, etc. in human or animals. Furthermore, it can be usefully used as a starter for fermentation.

Because the *Lactobacillus sakei* WIKIM0109 according to the present disclosure, which is a lactic acid bacterium isolated form kimchi, exhibits an activity of inhibiting IgG and IgG2a production in blood and inhibiting IFN-_{Y}, IL-17 and TNF-α production, it can be utilized variously for prevention or amelioration of arthritis. Furthermore, it can be usefully used as a starter for fermentation.

### [Brief Description of Drawings]

FIG. 1 shows the incidence of arthritis and arthritic score in a mouse model to which the strain of the present disclosure was administered orally.
FIG. 2 shows a result of measuring the paw thickness of an arthritis-induced mouse model to which the strain of the present disclosure was administered orally.
FIG. 3 shows a result of measuring IgG and collagen-specific IgG (CII-IgG) production in blood.
FIG. 4 shows a result of measuring IgG2a and collagen-specific IgG2a (CII-IgG2a) production in blood.
FIG. 5 shows a result of measuring collagen antigen-specific IFN-γ, IL-17 and TNF-α production.
FIG. 6 shows a result of comparing immunoregulatory capacity with LS35 and LS41, which are *Lactobacillus sakei* strains different from WIKIM0109.

### [Best Mode]

Hereinafter, the present disclosure is described in detail through examples. The following examples are for illustrative purposes only and the scope of the present disclosure is not limited by the examples.

### [Examples]

### Example 1: Isolation and identification of strain

A bacterial single colony obtained by smearing a crude liquid extract of kimchi in MRS culture medium was collected and cultured in MRS broth. DNA was extracted using the QIAamp DNA Mini Kit (Qiagen, Germany). The extracted DNA was identified using 1% agarose gel. PCR was conducted using the extracted genomic DNA as a template to amplify the 16S rRNA gene. The PCR condition was 30 cycles of denaturation at 95 °C for 1 minute, annealing at 45 °C for 1 minute and extension at 72 °C for 1 minute 30 seconds. The sequence of the obtained PCR product was analyzed by Macrogen (Seoul, Korea). Bacterial identification was performed by similarity analysis of the 16S rRNA sequence using the Basic Local Alignment Search Tool (BLAST) search engine of the National Center for Biotechnology Information (NCBI, www.ncbi.nlm.nih.gov).

As a result of the 16S rRNA base sequence analysis for identification of the microorganism, the strain isolated in this example was found to have a nucleic acid sequence of SEQ ID NO: 1.

The microorganism of the present disclosure was named *Lactobacillus sakei* WIKIM0109 and deposited on March 7, 2019 in the Korean Collection for Type Cultures (accession number: KCTC13818BP).

### Example 2: Confirmation of arthritis-ameliorating effect of Lactobacillus sakei WIKIM0109

The *Lactobacillus sakei* WIKIM0109 strain isolated in Example 1 was cultured in MRS medium at 30 °C for 24 hours, and the cultured bacteria were centrifuged at 8,000 rpm for 5 minutes and then washed with PBS to remove the remaining medium components. Then, the number of the bacteria was quantified to be 1×10¹⁰CFU/mL using PBS, and 0.1 mL (1×10⁹ CFU) was orally administered to a collagen-induced arthritis mouse model using a sonde, five times a week. Sterile PBS was administered to negative and positive control groups.

### 1) Measurement of incidence of arthritis and arthritic score in collagen-induced arthritis mouse model

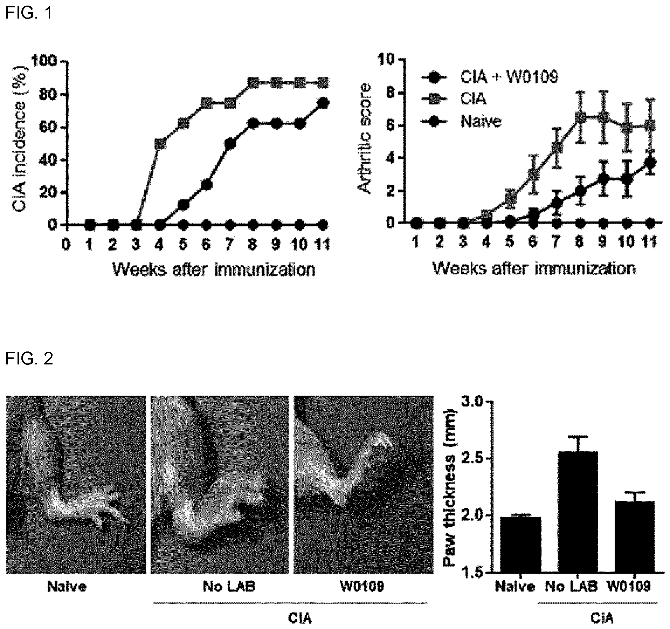

Arthritic score and incidence of arthritis for 12 weeks during which the *Lactobacillus sakei* WIKIM0109 strain was orally administered to the collagen-induced arthritis mouse model are shown in FIG. 1.

As a result, the collagen-induced arthritis control group (CIA-No LAB) showed the highest incidence of arthritis and arthritic score, and the incidence of arthritis and arthritic score were decreased significantly in the *Lactobacillus sakei* WIKIM0109 treatment group (WIKIM0109).

### 2) Sensory evaluation and measurement of paw thickness

After orally administering the *Lactobacillus sakei* WIKIM0109 strain to experimental animals for 4 weeks, the condition of arthritis was evaluated by clinical visual evaluation and paw thickness was measured.

As shown in FIG. 2, symptoms such as erythema, swelling, etc. were observed in the joint and paw of the collagen-induced arthritis control group (CIA-No LAB) in contrast to the normal group (Naive). Also, it was confirmed that the erythema and joint swelling were ameliorated and the paw thickness was decreased in the *Lactobacillus sakei* WIKIM0109 treatment group (WIKIM0109) as compared to the control group.

### 3) Measurement of concentrations of IgG and IqG2a in blood

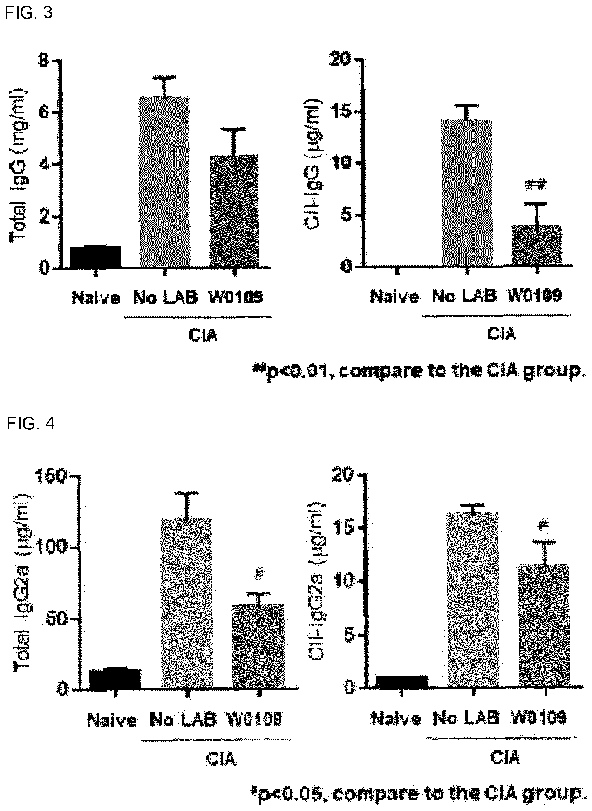

After taking blood from the experimental animals, the concentrations of IgG, collagen antigen-specific IgG, IgG2a and collagen antigen-specific IgG2a in blood were measured. FIG. 3 shows a result of measuring the concentrations of IgG and collagen antigen-specific IgG (CII-IgG), and FIG. 4 shows a result of measuring the concentrations of IgG2a and collagen antigen-specific IgG2a (CII-IgG2a).

**[Table 1]**

| | Before administration | After administration | Inhibition(%) |
|---|---|---|---|
| IgG | 6.52 ± 0.81 mg/ml | 4.796 ± 1.08 mg/ml | 26.5 |
| CII-IgG | 14.04 ± 1.51 *µ*g/ml | 3.77 ± 2.21 *µ*g/ml | 73.2 |
| IgG2a | 118.2 ± 19.55 *µ*g/ml | 57.65 ± 9.02 *µ*g/ml | 51.3 |
| CII-IgG2a | 16.17 ± 0.88 *µ*g/ml | 11.26 ± 2.34 *µ*g/ml | 30.4 |

As seen from FIG. 3 and Table 1, the production of IgG and IgG2a in blood was induced by the arthritis induction as compared to the normal group. It was also confirmed that the administration of the *Lactobacillus sakei* WIKIM0109 to the experimental animals resulted in decreased concentrations of IgG and IgG2a. In particular, the production of collagen antigen-specific IgG2a (CII-IgG2a) was significantly inhibited by about 30.4% as compared to the control group.

### 4) Measurement of production of collagen antigen-specific IFN-γ, IL-17 and TNF-α

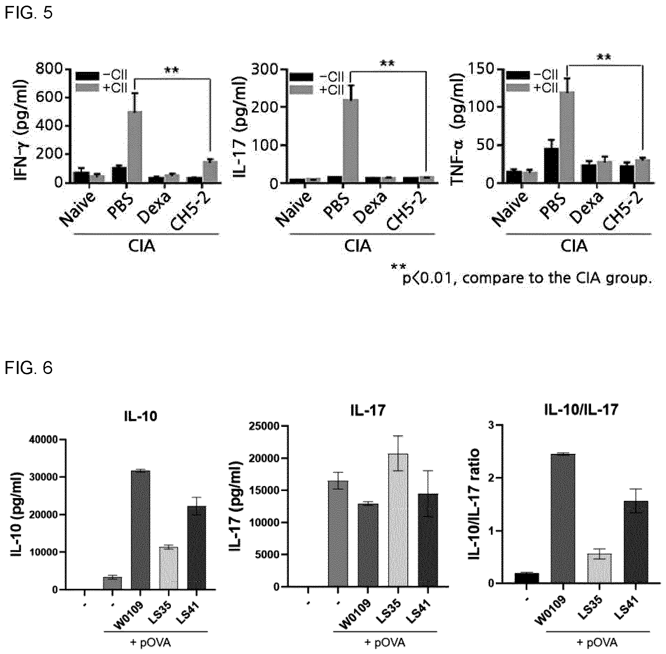

After obtaining single cells from the lymph nodes around the joint of the experimental animals, 5×10⁵ cells were dispensed onto a 96-well plate. After treating with collagen (50 µg/mL) for 48 hours, the concentrations of antigen-specific IFN-_{Y}, IL-17 and TNF-α were measured from the supernatant. As seen from FIG. 5, the production of antigen-specific IFN-γ, IL-17 and TNF-α was increased in the arthritis-induced group as compared to the normal group. It was also confirmed that the production of antigen-specific IFN-_{Y}, IL-17 and TNF-α was significantly decreased when the *Lactobacillus sakei* WIKIM0109 was administered to experimental animals.

### 5) Comparative experiments of WIKIM0109 with other strains of Lactobacillus sakei

In order to investigate whether the effect of the *Lactobacillus sakei* WIKIM0109 is common to *Lactobacillus sakei,* (1) the increase in the production of the immune-suppressing cytokine IL-10 and (2) the decrease in the production of the immune-enhancing cytokine IL-17 were compared with *Lactobacillus sakei* LS35 and LS41 acquired from the World Institute of Kimchi, an annex of the Korea Food Research Institute.

*Lactobacillus sakei* LS35 was isolated from watery kimchi and *Lactobacillus sakei* LS41 were isolated from pa-gat kimchi. They were statically cultured in a 30 °C incubator for 24 hours using Lactobacilli MRS (BD Difco) liquid medium. After sequencing of the gene encoding 16S rRNA, they were identified as *Lactobacillus sakei* through homology analysis using the Basic Local Alignment Search Tool (BLAST) of the National Center for Biotechnology Information (NCBI).

OTII mouse having OVA-specific T cells were purchased from Jackson Laboratory and used after breeding. 5×10⁵ single splenocytes of the OTII mouse were dispensed onto a 96-well plate and cultured at 37 °C for 72 hours after treating with OVA peptide (OVA323-339) and kimchi lactic acid bacteria at MOI 1. The amount of IL-17 and IL-10 present in the culture medium was measured using the Cytometric Bead Assay (CBA) mouse Th1/Th2/Th17 kits purchased from BD Bioscience. The production of the cytokines was analyzed based on standard curves by using FACSCanto II (BD Bioscience).

As a result, it was confirmed that the WIKIM0109 strain resulted in the highest production of IL-10, the lowest production of IL-17 and the highest IL-10/IL-17 ratio among the different strains of *Lactobacillus sakei* (FIG. 6).

Although the LS41 strain also increased the production of the immune-suppressing cytokine IL-10, the WIKIM0109 strain showed about 40% or more production of IL-10. The production by the LS35 strain was only about 1/3 of that of the WIKIM0109 strain.

For the immune-enhancing cytokine IL-17, although the LS41 strain did not show significant decrease in production, the WIKIM0109 strain significantly decreased the production of IL-17. On the contrary, the LS35 strain increased the production of IL-17.

Although the specific exemplary embodiments of the present disclosure have been described in detail, it will be obvious to those having ordinary knowledge in the art that they are merely preferred exemplary embodiments and the scope of the present disclosure is not limited thereto. Accordingly, it is to be understood that the substantial scope of the present disclosure is defined by the appended claims and their equivalents.

### [Deposition Number]

Depository agency: Korean Collection for Type Cultures (international). Accession number: KCTC13818BP.

Date of accession: Mar. 07, 2019.

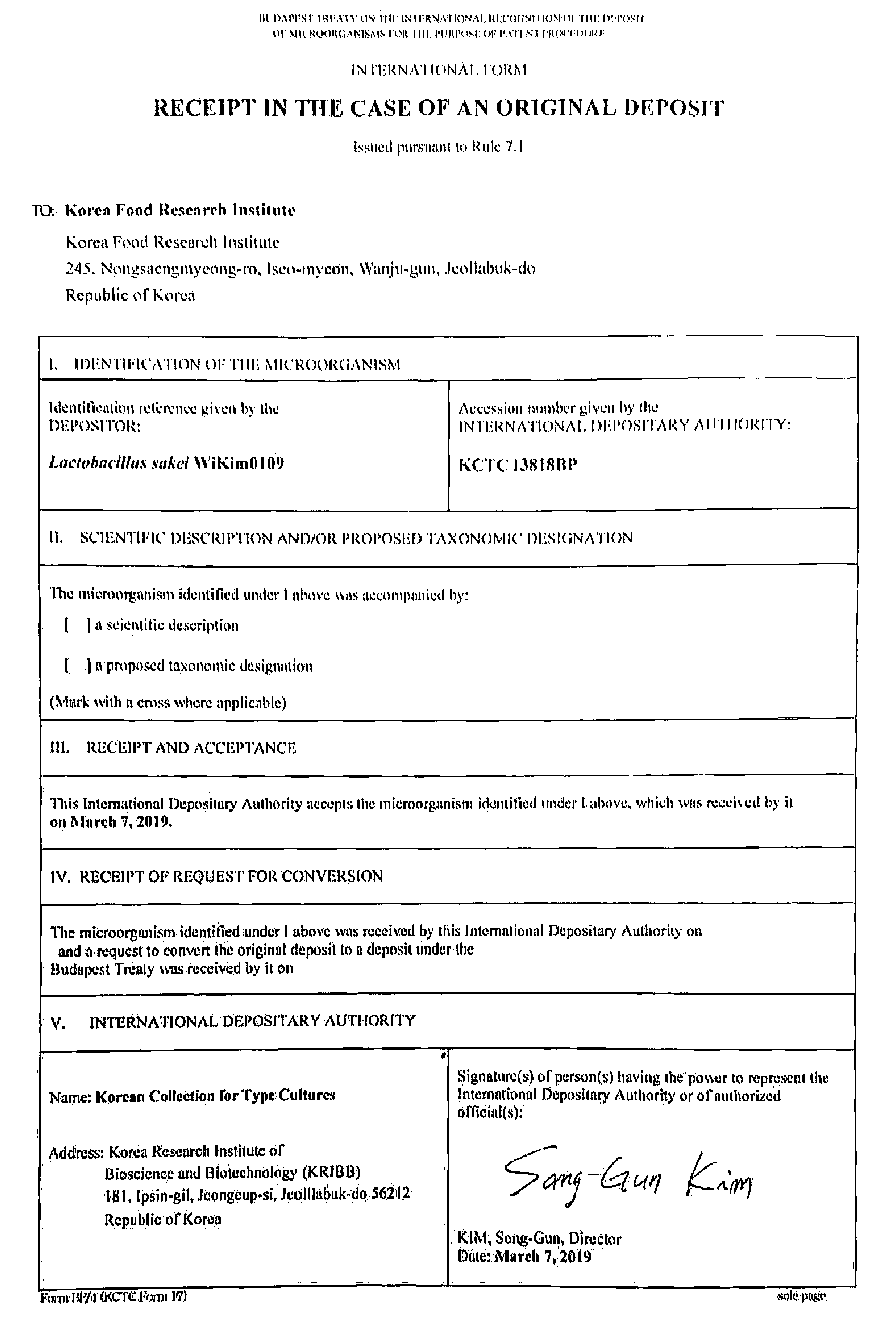
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Korea Food Research Institute
Korea Food Research Institute
245, Nongsaengmyeong-ro, Iseo-myeon, Wanju-gun, Jeollabuk-do
Republic of Korea

I. IDENTIFICATION OF THE MICROORGANISM
Identification reference given by the
DEPOSITOR: | Accession number given by the
INTERNATIONAL DEPOSITARY AUTHORITY:
Lactobacillus sakei WiKim0109 | KCTC 13818BP

II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)

III. RECEIPT AND ACCEPTANCE
This International Depositary Authority accepts the microorganism identified under I above, which was received by it
on March 7, 2019.

IV. RECEIPT OF REQUEST FOR CONVERSION
The microorganism identified under I above was received by this International Depositary Authority on
and a request to convert the original deposit to a deposit under the
Budapest Treaty was received by it on

V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: March 7, 2019

Form BP/4 (KCTC Form 17) sole page

<110>    Korea Food Research Institute

<120>    Lactobacillus sakei WIKIM0109 having anti-arthritis activity and
         composition for comprising the same

<130>    PCT677

<150>    KR 10-2019-0034211
<151>    2019-03-26

<160>    1

<170>    KoPatentIn 3.0

<210>    1
<211>    1409
<212>    DNA
<213>    Unknown

<220>
<223>    Lactobacillus sakei


<400>    1
agtcgaacgc actctcgttt agattgaagg agcttgctcc tgattgataa acatttgagt        60

gagtggcgga cgggtgagta acacgtgggt aacctgccct aaagtggggg ataacatttg       120

gaaacagatg ctaataccgc ataaaaccta acaccgcatg gtgtagggtt gaaagatggt       180

ttcggctatc actttaggat ggacccgcgg tgcattagtt agttggtgag gtaaaggctc       240

accaagaccg tgatgcatag ccgacctgag agggtaatcg gccacactgg gactgagaca       300

cggcccagac tcctacggga ggcagcagta gggaatcttc cacaatggac gaaagtctga       360

tggagcaacg ccgcgtgagt gaagaaggtt ttcggatcgt aaaactctgt tgttggagaa       420

gaatgtatct gatagtaact gatcaggtag tgacggtatc caaccagaaa gccacggcta       480

actacgtgcc agcagccgcg gtaatacgta ggtggcaagc gttgtccgga tttattgggc       540

gtaaagcgag cgcaggcggt ttcttaagtc tgatgtgaaa gccttcggct caaccgaaga       600

agtgcatcgg aaactgggaa acttgagtgc agaagaggac agtggaactc catgtgtagc       660

ggtgaaatgc gtagatatat ggaagaacac cagtggcgaa ggcggctgtc tggtctgtaa       720

ctgacgctga ggctcgaaag catgggtagc aaacaggatt agataccctg gtagtccatg       780

ccgtaaacga tgagtgctag gtgttggagg gtttccgccc ttcagtgccg cagctaacgc       840

attaagcact ccgcctgggg agtacgaccg caaggttgaa actcaaagga attgacgggg       900

gcccgcacaa gcggtggagc atgtggttta attcgaagca acgcgaagaa ccttaccagg       960

tcttgacatc ctttgaccac tctagagata gagctttccc ttcggggaca aagtgacagg      1020

tggtgcatgg ttgtcgtcag ctcgtgtcgt gagatgttgg gttaagtccc gcaacgagcg 1080

caacccttat tactagttgc cagcatttag ttgggcactc tagtgagact gccggtgaca 1140

aaccggagga aggtggggac gacgtcaaat catcatgccc cttatgacct gggctacaca 1200

cgtgctacaa tggatggtac aacgagttgc gagaccgcga ggtttagcta atctcttaaa 1260

accattctca gttcggattg taggctgcaa ctcgcctaca tgaagccgga atcgctagta 1320

atcgcggatc agcatgccgc ggtgaatacg ttcccgggcc ttgtacacac cgcccgtcac 1380

accatgagag tttgtaacac ccaaagccg 1409

## Claims

1. *Lactobacillus sakei* WIKIM0109 with an accession number KCTC13818BP.

2. A pharmaceutical composition for preventing or treating inflammatory arthritis, comprising *Lactobacillus sakei* WIKIM0109 (accession number: KCTC13818BP), a culture thereof, a lysate thereof or an extract thereof as an active ingredient.

3. The pharmaceutical composition according to claim 2, wherein the inflammatory arthritis is rheumatoid arthritis, ankylosing spondylitis, osteoarthritis or psoriatic arthritis.

4. The pharmaceutical composition according to claim 2, wherein the composition reduces hypersensitive inflammatory response by inhibiting IgG and IgG2a production in blood.

5. The pharmaceutical composition according to claim 2, wherein the composition reduces hypersensitive inflammatory response by inhibiting collagen antigen-specific IFN-_{Y}, IL-17 and TNF-α production.

6. The pharmaceutical composition according to claim 2, wherein the composition is a composition for oral administration.

7. A food composition for preventing and ameliorating inflammatory arthritis, comprising *Lactobacillus sakei* WIKIM0109 (accession number: KCTC13818BP), a culture thereof, a lysate thereof or an extract thereof as an active ingredient.

8. The food composition according to claim 7, wherein the inflammatory arthritis is rheumatoid arthritis, ankylosing spondylitis, osteoarthritis or psoriatic arthritis.

9. The food composition according to claim 7, wherein the composition reduces hypersensitive inflammatory response by inhibiting IgG and IgG2a production in blood.

10. The food composition according to claim 7, wherein the composition reduces hypersensitive inflammatory response by inhibiting collagen antigen-specific IFN-γ, IL-17 and TNF-α production.

11. The food composition according to claim 7, wherein the food is a functional health food.

12. The food composition according to claim 7, wherein the food is a beverage, a bar or a fermented milk.

13. A composition for enhancing immunity, comprising *Lactobacillus sakei* WIKIM0109 (accession number: KCTC13818BP), a culture thereof, a lysate thereof or an extract thereof as an active ingredient.

14. A composition for intestinal regulation, comprising *Lactobacillus sakei* WIKIM0109 (accession number: KCTC13818BP), a culture thereof, a lysate thereof or an extract thereof as an active ingredient.

15. A lactic acid bacterium starter for fermenting a food or a feed, comprising *Lactobacillus sakei* WIKIM0109 (accession number: KCTC13818BP), a culture thereof, a lysate thereof or an extract thereof as an active ingredient.

16. A feed or feed additive composition, comprising *Lactobacillus sakei* WIKIM0109 (accession number: KCTC13818BP), a culture thereof, a lysate thereof or an extract thereof as an active ingredient.
